(19) Europäisches Patentamt
European Patent Office
Office européen des brevets

(11) Numéro de publication: **0 170 937 B1**

# (12) FASCICULE DE BREVET EUROPEEN

(45) Date de publication du fascicule du brevet: **03.08.88**

(51) Int. Cl.⁴: **C 07 H 15/26**, C 07 D 233/88, A 61 K 31/70

(21) Numéro de dépôt: **85108800.5**

(22) Date de dépôt: **13.07.85**

(54) **Sulfate double de desoxyfructosyl-sérotonine et de créatinine, sa préparation et médicament le contenant.**

(30) Priorité: **09.08.84 CH 3823/84**

(43) Date de publication de la demande: **12.02.86 Bulletin 86/7**

(45) Mention de la délivrance du brevet: **03.08.88 Bulletin 88/31**

(84) Etats contractants désignés: **AT BE DE FR GB IT LU NL SE**

(56) Documents cités:
**CHEMICAL ABSTRACTS, vol. 94, no. 7, 16 février 1981, page 102, no. 41940r, Columbus, Ohio, US; P. JAYARAMAN et al.: "Inhibition of the incorporation of [3H]DOPA in mycobacterium leprae by desoxyfructoserotonin"**
**CHEMICAL ABSTRACTS, vol. 76, no. 5, 31 janvier 1972, no. 25021q, Columbus, Ohio, US; I.Kh. FEL'DMAN et al.: "Serotonin-beta-14C-creatinine sulfate monohydrate"**

(73) Titulaire: **SOCIETE DES PRODUITS NESTLE S.A., Case postale 353, CH-1800 Vevey (CH)**

(72) Inventeur: **Bertholet, Raymond, Chemin Vers-chez-Cochard 11, CH-1807 Blonay (CH)**
Inventeur: **Hirsbrunner, Pierre, Chemin des Romains, CH-1801 Les Monts-de-Corsier (CH)**

EP 0 170 937 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

L'invention concerne le sulfate double de désoxyfructosyl-sérotonine et de créatinine, un procédé de préparation de celui-ci et un médicament le contenant.

Le brevet français N° 2317937 concerne de nouveaux dérivés de la sérotonine (5-hydroxy-tryptamine), notamment l'oxalate de 1-désoxy-(5-hydroxy-tryptamino)-D-fructose ou désoxyfructosyl-sérotonine, ci-après «DFS» obtenus par réarrangement d'Amadori. Dans ce brevet, la DFS est décrite comme médicament contre l'agrégation plaquettaire et radioprotecteur. Plus récemment, la DFS s'est révélée très active dans le traitement de la lèpre (Jayaraman P., Mahadevan P.R., Mester L., Mester M., Biochemical Pharmacology, Vol. 29, 2526–28, 1980).

On sait que pour l'utilisation à titre de médicament, il est nécessaire que la substance active soit présentée sous forme unitaire, cristalline et stable. Or, la DFS est instable: elle se présente sous forme d'un produit blanc et amorphe, non cristallisable, brunissant après environ un jour à la température ordinaire en formant des polymères. L'oxalate de DFS est également amorphe, de couleur jaune, présente des impuretés et brunit à l'entreposage, ce qui exclut son utilisation comme médicament. Les techniques de cristallisation usuelles par les solvants, par exemple par l'alcool, l'application du froid, l'addition d'amorces à la cristallisation, etc. n'ont pas permis son obtention en phase solide à cause de sa très forte hydrosolubilité.

L'invention se rapporte au sulfate double de 1-désoxy-(5-hydroxy-tryptamino)-D-fructose et de 1-méthyl-hydantoïne-2-imide (créatinine) sous forme cristalline ne présentant pas les défauts des composés décrits.

Ce sel est soluble dans les milieux aqueux à froid et très soluble dans ceux-ci à chaud. Le pH d'une solution aqueuse à 1% en poids étant 3,5, les fonctions amine secondaire et imine respectivement de la DFS et du 1-méthyl-hydantoïne-2-imide sont protonées, ce qui permet d'attribuer au sel la formule suivante:

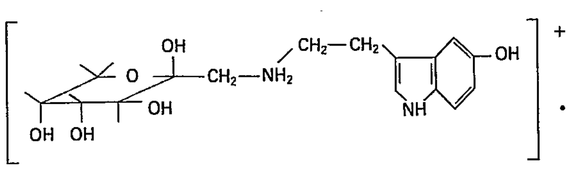

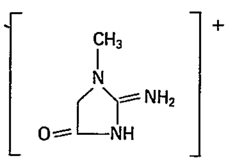

• $SO_4^{2-}$ • $2H_2O$ (I)

désigné dans la suite de l'exposé par «DFSCS».

Une étude au microscope optique et polarisante a montré que cette nouvelle phase solide se présentant sous la forme d'une poudre cristalline blanche était composée de cristaux à caractère fortement biréfringent et à basse symétrie de type mono- ou triclinique.

L'analyse chimique a permis d'établir la composition des cristaux conduisant par application de la loi des proportions simples à un sulfate double.

De poids moléculaire 585,6 et de zone de fusion 136–140 °C (avec décomposition) il a une densité apparente d'environ 30 g/100 ml et un pouvoir rotatoire $[\alpha]_D^{20}$ = 20,0 à –21,0° (c=1, eau).

Il est insoluble dans les alcools à 2 atomes de carbone et plus, les éthers, les esters, les cétones et les solvants halogénés, légèrement soluble dans le méthanol, soluble dans le diméthylsulfoxyde.

Il se caractèrise par une stabilité remarquable en phase solide: entreposé 6 mois à 45 °C sous forme de gélules en emballage de verre, aucune altération n'a été constatée.

L'invention concerne également un procédé de préparation de DFSCS, caractérisé par le fait qu'on ajoute du 1-méthyl-hydantoïne-2-imide, à une solution aqueuse de DFS contenant de l'acide sulfurique à un pH d'environ 3 et qu'on sépare le DFSCS du milieu réactionnel.

De préférence, on effectue la séparation par concentration de la solution aqueuse, addition d'un solvant miscible à l'eau dans lequel le DFSCS est insoluble et cristallisation du DFSCS.

On utilise de préférence un léger excès de créatinine par rapport à la DFS, par exemple 1,1 à 1,2 fois la quantité molaire de DFS. La solution peut être concentrée à environ la moitié de son volume, par exemple par évaporation sous pression réduite.

Comme solvant, on utilise avantageusement l'acétone ou les alcools ayant 1 à 4 atomes de carbone, de préférence l'éthanol.

On sépare alors les cristaux formés, par exemple par filtration, et on les sèche, par exemple sous vide. Après recristallisation éventuelle, par exemple dans le système eau/éthanol, filtration et séchage comme indiqué ci-dessus, on recueille le DFSCS sous forme de cristaux blancs.

La DFS utilisée comme produit de départ peut être obtenue par exemple par condensation du D-glucose avec la 5-hydroxytryptamine suivie d'un réarrangement d'Amadori (conversion de N-glycoside d'un aldose en N-glycoside du cétose correspondant en présence d'un catalyseur acide ou basique) selon les techniques connues (voir «Methods in Carbohydrate Chemistry», Vol. 2, Academic Press N.Y., 1963, p. 99).

On préfère utiliser un excès de D-glucose de 1,5 à 3 fois la quantité molaire de sérotonine engagée. Avantageusement, la sérotonine est présente sous la forme d'un de ses sels, par exemple comme hydrogénoacétate.

La réaction a lieu dans un milieu solvant anhydre en atmosphère inerte, par exemple sous azote, de manière à éviter l'hydrolyse de l'aldosylamine intermédiaire. Le solvant doit pouvoir solubiliser le glucose et la sérotonine. On utilise avantageusement un alcool inférieur c'est-à-dire ayant 1 à 4 atomes de carbone, par exemple le méthanol, l'éthanol ou l'isopropanol, le méthanol étant

préféré car il permet une meilleure sélectivité de la réaction.

La réaction est, de préférence, catalysée par les acides. Le catalyseur acide est choisi parmi les acides minéraux ou de préférence organiques qui permettent, suivant leur nature et la quantité ajoutée, de conférer au milieu réactionnel un pH compris entre 3 et 5 et de préférence de 4,2. On peut citer les acides mono- ou polycarboxyliques, par exemple l'acide formique, oxalique ou acétique, l'acide formique étant préféré.

La température va de l'ambiante à la température de reflux du solvant.

La réaction dure en général de 30 à 150 min. Suivant sa durée, la réaction est plus ou moins complète et conduit à un mélange comprenant en poids des espèces contenant de la sérotonine, de 60 à 83% de DFS, de 3 à 35% des sérotonine résiduelle et de 5 à 14% de produits secondaires. On trouve également dans le milieu réactionnel l'excès de glucose et l'acide introduit.

De préférence, on ajoute de l'eau au milieu réactionnel et on élimine le solvant, par exemple par distillation sous pression réduite. On décolore avantageusement la solution aqueuse par exemple avec du charbon actif et on ajoute la quantité d'acide sulfurique, de préférence concentré, permettant de conférer à la solution un pH d'environ 3.

Aussi bien la DFS hydrolysable en sérotonine que la sérotonine elle-même peuvent être recyclées mais pas les produits secondaires, c'est-à-dire les dérivés di-, tri-substitués et les polymères.

Dans une variante préférée, on interrompt la réaction après environ 40 min et, après avoir séparé le DFSCS comme indiqué ci-dessus, on extrait la sérotonine de la phase liquide par échange d'ions ou, de préférence, par solvant. Dans ce dernier cas, par exemple, on ajuste le pH de la phase liquide au point isoélectrique de la sérotonine, par addition d'une base forte, par exemple l'hydroxyde de sodium ou de potassium, soit à environ 10,8 et on extrait celle-ci par un alcool aliphatique ayant 4 à 8 atomes de carbone, par exemple l'isobutanol ou un alcool benzylique (c'est-à-dire benzylique ou méthylbenzylique) puis, après élimination du solvant, on recycle la sérotonine en amont de la réaction de formation de la DFS. Avantageusement, on met la sérotonine à recycler sous la forme du sel qui a servi à la réaction. Ainsi, on neutralise la solution alcoolique, par exemple isobutanolique jusqu'à un pH d'environ 6 avec, par exemple, l'acide acétique et on élimine le solvant, par exemple par évaporation sous pression réduite.

Selon un mode de réalisation préféré du procédé de l'invention, pour préparer la solution aqueuse de DFS contenant de l'acide sulfurique, on ajoute au milieu réactionnel contenant la DFS en présence d'eau un excès d'hydroxyde de calcium par rapport à la DFS, on recueille un complexe d'addition insoluble, on le traite avec un acide précipitant le calcium, on élimine le calcium sous forme de sel insoluble, on recueille la DFS en solution et on ajoute de l'acide sulfurique à la solution.

En ajoutant l'hydroxyde de calcium, de préférence en suspension aqueuse ou hydroalcoolique sous agitation à température ambiante dans des proportions molaires DFS/$Ca(OH)_2$ de 1:3 à 1:4, on forme un complexe d'addition du type «sucrate». De préférence, on ajoute également à la suspension un réducteur, par exemple le dithionite de sodium. Après environ 10 min, on filtre la phase solide et on la lave à l'eau.

Avantageusement, on récupère la sérotonine se trouvant dans les eaux mères. Pour ce faire, on ajoute de l'acide sulfurique jusqu'à ce que la solution ait un pH d'environ 3, on élimine le sulfate de calcium formé, par exemple par filtration, on élimine la majeure partie de l'eau, par exemple par évaporation sous pression réduite et, après avoir ajusté le pH de la solution à environ 10,8, on effectue les opérations d'extraction par un alcool aliphatique ayant 4 à 8 atomes de carbone ou un alcool benzylique et de formation du sel désiré de la sérotonine comme indiqué ci-dessus.

On met le complexe insoluble DFS.$Ca(OH)_2$, constituant la phase solide mentionnée ci-dessus, en suspension aqueuse. Le pH de la suspension est fortement alcalin, de 12 à 13.

On précipite le calcium sous forme d'un sel par traitement du complexe d'addition en suspension aqueuse avec un acide approprié. Le choix de l'acide est dicté par le fait que celui-ci forme avec le calcium un sel insoluble dans les milieux aqueux. On peut utiliser un acide organique ou minéral, par exemple l'acide oxalique, citrique, tartrique ou l'acide phosphorique ou de préférence sulfurique, de préférence concentré, on précipite les sels de calcium insolubles correspondant aux acides engagés, par exemple $CaSO_4.2H_2O$ (gypse) dans le cas de l'acide sulfurique.

Après séparation de la phase solide, par exemple par filtration, on trouve en solution par exemple l'oxalate, citrate, tartrate ou encore l'hydrogénophosphate ou, de préférence, le sulfate neutre de DFS.

Selon un mode de réalisation préféré du procédé selon l'invention, dans lequel on a préparé le sulfate neutre de DFS en solution, on ajoute à la solution un léger excès molaire de créatinine et de l'acide sulfurique comme indiqué précédemment et on obtient par cristallisation à l'alcool le DFSCS.

Après séchage de la phase solide et recristallisation éventuelle, par exemple dans un système eau/alcool, le sel obtenu contient environ 90% de la quantité théorique de DFS. Calculé par rapport à la sérotonine engagée, le rendement est d'environ 60%.

De préférence on ajoute aux eaux mères, dont on a éliminé l'alcool par évaporation, de l'hydroxyde de calcium, on sépare le complexe insoluble par exemple par filtration et on le recycle en l'incorporant à la charge suivante dans l'étape d'addition de l'hydroxyde de calcium. Le recyclage du complexe DFS.$Ca(OH)_2$ permet d'augmenter le rendement à environ 70%.

En récupérant la sérotonine à partir des eaux mères après la précipitation du complexe DFS.$Ca(OH)_2$, celui-ci est d'environ 80%. Par comparaison, l'oxalate brut de DFS est obtenu sous forme amorphe selon le brevet français N° 2317937 avec un rendement d'environ 25%.

L'invention concerne également un médicament caractérisé en ce qu'il contient comme principe actif le DFSCS.

La formulation d'un tel médicament est adaptée au mode d'administration.

Administré par exemple par voie orale ou entérale, il peut se présenter sous forme de sirop, capsule, gélule, comprimé ou dragée.

Destiné par exemple à l'administration par voie parentérale, il peut se présenter sous forme d'une solution ou suspension, stabilisée physiquement et chimiquement, stérile et apyrogène.

Adapté par exemple à l'administration par voie topique, il peut se présenter sous forme de lotion, pommade, lait, crème ou gel.

La concentration du principe actif peut être 10 à 50% en poids.

Par exemple une dose quotidienne de 400–450 mg ingérée sous forme de gélules convient pour le traitement de la lèpre.

Les exemples suivants illustrent l'invention. Dans ceux-ci les pourcentages et parties sont pondéraux sauf indication contraire.

Exemple 1

On chauffe à reflux (60 °C) 2,36 g d'hydrogénoacétate de sérotonine (10 mmoles), 3,6 g de D-glucose anhydre (20 mmoles), 0,65 g d'acide formique et 75 ml de méthanol anhydre sous azote pendant 150 min. On ajoute 50 ml d'eau et on élimine le méthanol par distillation sous vide. On décolore la solution aqueuse à l'aide de 1,5 g de charbon actif. Après filtration, on ajoute 0,9 g de créatinine (8 mmoles) et 2,8 g d'acide sulfurique à 30% pour ajuster le pH à 3, puis on concentre. On obtient ainsi 33 g d'une solution de coloration orangée à laquelle on ajoute lentement 75 ml d'éthanol à 96%. Une phase cristalline apparaît; on sépare celle-ci par filtration puis on la lave avec de l'éthanol à 96%. Après séchage à 40 °C sous vide, on obtient 2,45 g de cristaux de coloration légèrement beige. Par recristallisation dans le système eau/éthanol, filtration et séchage, on recueille 2,12 g de cristaux blancs de formule I indiquée précédemment.

La structure a été vérifiée par spectroscopie R.M.N. du proton et du carbone et par l'analyse chimique:

| | analyse (% en poids) | théorie (% en poids) |
|---|---|---|
| Azote total | 12,12 | 11,97 |
| $H_2O$ (Méthode K. Fischer) | 6,48 | 6,15 |
| Créatinine | 19,8 | 19,32 |
| $H_2SO_4$ (Méthode acidimétrique courbe de titration METROHM®) | 16,75 | 17,05 |

Exemple 2

A 600 g de méthanol, on ajoute 23,6 g d'hydrogénoacétate de sérotonine (0,1 mole), 27,0 g de D-glucose anhydre et 6,5 g d'acide formique. Ce mélange est porté au reflux pendant 120 min sous atmosphère inerte. Après ce traitement, la totalité de la sérotonine a réagi pour former 25 g de DFS plus des produits secondaires. On ajoute 300 g d'eau au mélange réactionnel, puis on distille le méthanol sous vide. Dans la solution aqueuse acide (pH 3,9) obtenue, on ajoute 2,5 g de dithionite de sodium et 30 g d'hydroxyde de calcium (en suspension dans 50 g d'eau). Une phase solide apparaît. On agite le mélange (pH 12,2) pendant 10 min puis on le filtre. On lave la phase solide recueillie, qui est composée du complexe DFS.$Ca(OH)_2$ et d'un excès de $Ca(OH)_2$, à l'eau. Dans le filtrat se trouvent les impuretés et les sels.

On met la phase solide obtenue précédemment en suspension dans 250 g d'eau. On ajoute 95 g d'acide sulfurique à 30% et on agite pendant 60 min. Ce traitement libère la DFS de son complexe et précipite le calcium sous forme de gypse ($CaSO_4 \cdot 2\ H_2O$) qu'on sépare par filtration. On décolore le filtrat (pH 6) contenant la DFS sous forme de sulfate neutre à l'aide de 5 g de charbon actif sous agitation. Après filtration on obtient 400 g d'une solution de coloration orange à laquelle on ajoute 9,0 g de créatinine (0,08 mole) puis 17 g d'acide sulfurique de DFSCS sous vide jusqu'à un poids de 170 g puis on ajoute lentement et sous agitation 360 g d'éthanol à 96%. Une phase cristalline apparaît et après 1 h d'agitation à température ambiante, on filtre les cristaux formés, on les lave avec 150 g d'éthanol à 85% puis on les sèche à 40 °C sous vide. On recueille ainsi 37,0 g de cristaux légèrement beiges.

On concentre l'eau mère de cristallisation et on traite la phase aqueuse résiduelle qui contient encore 6,5 g de DFS comme précédemment. Pour ce faire, 6 g de $Ca(OH)_2$, puis 17 g d'acide sulfurique à 30% sont nécessaires. Après concentration du filtrat et adjonction de 2 g de créatinine, on cristallise à pH 3, encore 5,0 g de cristaux légèrement beiges.

Le total obtenu est de 42,0 g. Ce produit a une teneur en DFS de 52,5%. Par recristallisation dans le système eau/éthanol, on obtient 37,1 g de cristaux blancs d'une teneur en DFS de 55,4% et contenant 20,8% de créatinine.

Le rendement en DFS est de 20,5 g soit 60,6% par rapport à la sérotonine engagée.

Les analyses confirment la formule mentionnée précédemment.

Exemple 3

On répète les opérations de l'exemple 2 mais en réduisant la durée du reflux à 40 min. A ce stade de la réaction, seul 80% de la sérotonine engagée a réagi pour former 27 g de DFS et des impuretés. Le traitement à l'hydroxyde de calcium permet de séparer la DFS de la sérotonine n'ayant pas réagi, celle-ci restant soluble dans le filtrat et pouvant être extraite.

A cet effet, on concentre le filtrat sous vide jusqu'à un volume de 150 ml, puis on l'acidifie à pH 3 avec de l'acide sulfurique à 30%. On sépare le sulfate de calcium formé ($CaSO_4.2\ H_2O$) par filtration. On ajuste le pH du filtrat dans lequel se trouve la sérotonine à 10,8 (point isoélectrique de la sérotonine), par addition d'hydroxyde de sodium, puis on extrait celle-ci par 3×100 ml d'isobutanol. Après élimination de la phase aqueuse on réunit les 3 extraits isobutanoliques, on les neutralise à pH 6 avec de l'acide acétique, puis on les concentre sous vide. On receuille ainsi 4,0 g d'hydrogénoacétate de sérotonine, ce qui représente 16,6% de la sérotonine engagée.

Quant à la phase solide (le complexe DFS.$Ca(OH)_2$), elle fournira, après les différents traitements de l'exemple 2, 45 g de cristaux blancs d'une teneur en DFS de 52%. Le rendement en DFS est de 69%. Le recyclage de la sérotonine conduit à augmenter le rendement à 81%.

**Revendications pour les états contractants BE, DE, FR, GB, IT, LU, NL, SE**

1. Sulfate double de 1-désoxy-(5-hydroxy-tryptamino)-D-fructose et de 1-méthyl-hydantoïne-2-imide.

2. Médicament, caractérisé en ce qu'il contient comme principe actif le composé selon la revendication 1.

3. Procédé de préparation du composé selon la revendication 1, caractérisé par le fait qu'on ajoute du 1-méthyl-hydantoïne-2-imide à une solution aqueuse de 1-désoxy-(5-hydroxy-tryptamino)-D-fructose contenant de l'acide sulfurique à un pH d'environ 3 et qu'on sépare le sulfate double de 1-désoxy-(5-hydroxy-tryptamino)-D-fructose et de 1-méthyl-hydantoïne-2-imide du milieu réactionnel.

4. Procédé selon la revendication 3, caractérisé par le fait qu'on sépare le sulfate double de 1-désoxy-(5-hydroxy-tryptamino)-D-fructose par cristallisation à partir du milieu réactionnel en concentrant la solution et en ajoutant de l'éthanol à la solution concentrée.

5. Procédé selon la revendication 3, caractérisé par le fait que le 1-désoxy-(5-hydroxy-tryptamino)-D-fructose de départ est obtenu en faisant réagir le D-glucose en excès avec la 5-hydroxy-tryptamine dans un solvant anhydre en atmosphère inerte en présence d'acide oxalique ou formique conférant au milieu réactionnel un pH de 3 à 5.

6. Procédé selon la revendication 3, caractérisé par le fait qu'après avoir séparé le sulfate double de 1-désoxy-(5-hydroxy-tryptamino)-D-fructose, on traite le milieu réactionnel par une base forte de manière à ajuster le pH au point isoélectrique de la 5-hydroxy-tryptamine, on extrait cette dernière par un alcool aliphatique ayant 4 à 8 atomes de carbone ou un alcool benzylique, on élimine l'alcool et on recycle la 5-hydroxy-tryptamine en amont de la réaction de formation du 1-désoxy-(5-hydroxy-tryptamino)-D-fructose.

7. Procédé selon la revendication 3 ou 4, caractérisé par le fait que, pour préparer la solution aqueuse de 1-désoxy-(5-hydroxy-tryptamino)-D-fructose, contenant de l'acide sulfurique, on ajoute au milieu réactionnel contenant le 1-désoxy-(5-hydroxy-tryptamino)-D-fructose en présence d'eau un excès d'hydroxyde de calcium par rapport au 1-désoxy-(5-hydroxy-tryptamino)-D-fructose, on recueille un complexe d'addition insoluble, on le traite avec un acide précipitant le calcium, on élimine le calcium sous forme de sel insoluble, on recueille le 1-désoxy-(5-hydroxy-tryptamino)-D-fructose en solution et on ajoute de l'acide sulfurique à la solution.

8. Procédé selon la revendication 7, caractérisé par le fait que l'acide précipitant le calcium utilisé est l'acide sulfurique.

9. Procédé selon la revendication 7 ou 8, caractérisé par le fait qu'après la précipitation du complexe d'addition insoluble du 1-désoxy-(5-hydroxy-tryptamino)-D-fructose avec l'hydroxyde de calcium, on sépare celui-ci et on le lave, on recueille les eaux mères et de lavage, on les traite par l'acide sulfurique jusqu'à ce que la solution ait un pH d'environ 3, on élimine le sulfate de calcium formé, on ajuste le pH de la phase liquide au point isoélectrique de la 5-hydroxy-tryptamine par addition d'une base forte, on extrait la 5-hydroxy-tryptamine par un alcool aliphatique ayant 4 à 8 atomes de carbone ou un alcool benzylique, on élimine l'alcool et on recycle la 5-hydroxy-tryptamine en amont de la réaction de formation du 1-désoxy-(5-hydroxy-tryptamino)-D-fructose.

10. Procédé selon l'une des revendications 7 à 9, caractérisé par le fait qu'après séparation du sulfate double de 1-désoxy-(5-hydroxy-tryptamino)-D-fructose et de 1-méthyl-hydantoïne-2-imide, on ajoute de l'hydroxyde de calcium aux eaux mères, on sépare le complexe insoluble formé et on le recycle en l'incorporant à la charge suivante dans l'étape d'addition de l'hydroxyde de calcium.

**Revendications pour l'état contractant AT**

1. Procédé de préparation du sulfate double de 1-désoxy-(5-hydroxy-tryptamino)-D-fructose et de 1-méthyl-hydantoïne-2-imide, caractérisé par le fait qu'on ajoute du 1-méthyl-hydantoïne-2-imide à une solution aqueuse de 1-désoxy-(5-hydroxy-tryptamino)-D-fructose contenant de l'acide sulfurique à un pH d'environ 3 et qu'on sépare le sulfate double de 1-désoxy-(5-hydroxy-tryptamino)-D-fructose et de 1-méthyl-hydantoïne-2-imide du milieu réactionnel.

2. Procédé selon la revendication 1, caractérisé par le fait qu'on sépare le sulfate double de 1-désoxy-(5-hydroxy-tryptamino)-D-fructose par cristallisation à partir du milieu réactionnel en concentrant la solution et en ajoutant de l'éthanol à la solution concentrée.

3. Procédé selon la revendication 1, caractérisé par le fait que le 1-désoxy-(5-hydroxy-tryptamino)-D-fructose de départ est obtenu en faisant réagir le D-glucose en excès avec la 5-hydroxy-tryptamine dans un solvant anhydre en atmosphère inerte en présence d'acide oxalique ou formique conférant au milieu réactionnel un pH de 3 à 5.

4. Procédé selon la revendication 1, caractérisé par le fait qu'après avoir séparé le sulfate double de 1-désoxy-(5-hydroxy-tryptamino)-D-fructose, on traite le milieu réactionnel par une base forte de manière à ajuster le pH au point isoélectrique de la 5-hydroxy-tryptamine, on extrait cette dernière par un alcool aliphatique ayant 4 à 8 atomes de carbone ou un alcool benzylique, on élimine l'alcool et on recycle la 5-hydroxy-tryptamine en amont de la réaction de formation du 1-désoxy-(5-hydroxy-tryptamino)-D-fructose.

5. Procédé selon la revendication 1 ou 2, caractérisé par le fait que, pour préparer la solution aqueuse de 1-désoxy-(5-hydroxy-tryptamino)-D-fructose contenant de l'acide sulfurique, on ajoute au milieu réactionnel contenant le 1-désoxy-(5-hydroxy-tryptamino)-D-fructose en présence d'eau un excès d'hydroxyde de calcium par rapport au 1-désoxy-(5-hydroxy-tryptamino)-D-fructose, on recueille un complexe d'addition insoluble, on le traite avec un acide précipitant le calcium, on élimine le calcium sous forme de sel insoluble, on recueille le 1-désoxy-(5-hydroxy-tryptamino)-D-fructose en solution et on ajoute de l'acide sulfurique à la solution.

6. Procédé selon la revendication 5, caractérisé par le fait que l'acide précipitant le calcium utilisé est l'acide sulfurique.

7. Procédé selon la revendication 5 ou 6, caractérisé par le fait qu'après la précipitation du complexe d'addition insoluble du 1-désoxy-(5-hydroxy-tryptamino)-D-fructose avec l'hydroxyde de calcium, on sépare celui-ci et on le lave, on recueille les eaux mères et de lavage, on les traite par l'acide sulfurique jusqu'à ce que la solution ait un pH d'environ 3, on élimine le sulfate de calcium formé, on ajuste le pH de la phase liquide au point isoélectrique de la 5-hydroxy-tryptamine par addition d'une base forte, on extrait la 5-hydroxy-tryptamine par un alcool aliphatique ayant 4 à 8 atomes de carbone ou un alcool benzylique, on élimine l'alcool et on recycle la 5-hydroxy-tryptamine en amont de la réaction de formation du 1-désoxy-(5-hydroxy-tryptamino)-D-fructose.

8. Procédé selon l'une des revendications 5 à 7, caractérisé par le fait qu'après séparation du sulfate double de 1-désoxy-(5-hydroxy-tryptamino)-D-fructose et de 1-méthyl-hydantoïne-2-imide, on ajoute de l'hydroxyde de calcium aux eaux mères, on sépare le complexe insoluble formé et on le recycle en l'incorporant à la charge suivante dans l'étape d'addition de l'hydroxyde de calcium.

**Patentansprüche für die Vertragsstaaten BE, DE, FR, GB, IT, LU, NL, SE**

1. Doppelsulfat von 1-Desoxy-(5-hydroxy-tryptamino)-D-fructose und von 1-Methyl-hydantoin-2-imid.

2. Medikament, dadurch gekennzeichnet, dass es als Wirkstoff die Verbindung nach Anspruch 1 enthält.

3. Verfahren zur Herstellung einer Verbindung nach Anspruch 1, dadurch gekennzeichnet, dass man 1-Methyl-hydantoin-2-imid zu einer wässrigen Lösung von 1-Desoxy-(5-hydroxy-tryptamino)-D-fructose mit einem Gehalt an Schwefelsäure bei einem pH-Wert von ungefähr 3 zusetzt und das Doppelsulfat von 1-Desoxy-(5-hydroxy-tryptamino)-D-fructose und von 1-Methyl-hydantoin-2-imid aus dem Reaktionsmilieu abtrennt.

4. Verfahren nach Anspruch 3, dadurch gekennzeichnet, dass man das Doppelsulfat von 1-Desoxy-(5-hydroxy-tryptamino)-D-fructose durch Kristallisation aus dem Reaktionsmilieu abtrennt, indem man die Lösung konzentriert und zu der konzentrierten Lösung Ethanol zusetzt.

5. Verfahren nach Anspruch 3, dadurch gekennzeichnet, dass die eingesetzte 1-Desoxy-(5-hydroxy-tryptamino)-D-fructose durch Umsetzen von überschüssiger D-Glucose mit 5-Hydroxy-tryptamin in einem wasserfreien Lösungmittel unter inerter Atmosphäre in Gegenwart von Oxalsäure oder Ameisensäure, die dem Reaktionsmilieu einen pH-Wert von 3 bis 5 verleiht, erhalten wird.

6. Verfahren nach Anspruch 3, dadurch gekennzeichnet, dass nach dem Abtrennen des Doppelsulfats von 1-Desoxy-(5-hydroxy-tryptamino)-D-fructose das Reaktionsmilieu mit einer starken Base zur Einstellung des pH-Wertes auf den isoelektrischen Punkt des 5-Hydroxy-tryptamins behandelt wird, die letztgenannte Verbindung mit einem aliphatischen Alkohol mit 4 bis 8 Kohlenstoffatomen oder mit einem Benzylalkohol extrahiert wird, der Alkohol abgetrennt wird und das 5-Hydroxy-tryptamin in die Bildungsreaktion der 1-Desoxy-(5-hydroxy-tryptamino)-D-fructose zurückgeführt wird.

7. Verfahren nach Anspruch 3 oder 4, dadurch gekennzeichnet, dass zur Herstellung der wässrigen Lösung von 1-Desoxy-(5-hydroxy-tryptamino)-D-fructose mit einem Gehalt an Schwefelsäure zu einem die 1-Desoxy-(5-hydroxy-tryptamino)-D-fructose in Gegenwart von Wasser enthaltenden Reaktionsmilieu ein Calciumhydroxidüberschuss, bezogen auf 1-Desoxy-(5-hydroxy-tryptamino)-D-fructose, zugesetzt wird, ein unlöslicher Additionskomplex gewonnen wird, dieser mit einer das Calcium ausfällenden Säure behandelt wird, das Calcium in Form eines unlöslichen Salzes beseitigt wird, die 1-Desoxy-(5-hydroxy-tryptamino)-D-fructose in Lösung gewonnen wird und zu der Lösung Schwefelsäure zugesetzt wird.

8. Verfahren nach Anspruch 7, dadurch gekennzeichnet, dass die das Calcium ausfällende verwendete Säure die Schwefelsäure ist.

9. Verfahren nach Anspruch 7 oder 8, dadurch gekennzeichnet, dass nach dem Ausfällen des unlöslichen Additionskomplexes der 1-Desoxy-(5-hydroxy-tryptamino)-D-fructose mit dem Calciumhydroxid dieser Komplex abgetrennt und gewaschen wird, die Mutterlaugen der Waschung aufgefangen werden, diese mit Schwefelsäure behandelt werden, bis die Lösung einen pH-Wert von ungefähr 3 aufweist, das gebildete Calciumsulfat beseitigt wird, der pH-Wert der flüssigen Phase durch Zugabe einer starken Base auf den

isoelektrischen Punkt des 5-Hydroxy-tryptamins eingestellt wird, das 5-Hydroxy-tryptamin mit einem aliphatischen Alkohol mit 4 bis 8 Kohlenstoffatomen oder mit einem Benzylalkohol extrahiert wird, der Alkohol beseitigt wird und das 5-Hydroxy-tryptamin in die Bildungsreaktion der 1-Desoxy-(5-hydroxy-tryptamino)-D-fructose zurückgeführt wird.

10. Verfahren nach einem der Ansprüche 7 bis 9, dadurch gekennzeichnet, dass nach dem Abtrennen des Doppelsulfats von 1-Desoxy-(5-hydroxy-tryptamino)-D-fructose und von 1-Methyl-hydantoin-2-imid zu den Mutterlaugen Calciumhydroxid zugesetzt wird, der gebildete unlösliche Komplex abgetrennt wird und dieser zurückgeführt wird, indem er in die nächste Charge in der Calciumhydroxid-Zugabestufe aufgenommen wird.

**Patentansprüche für den Vertragsstaat AT**

1. Verfahren zur Herstellung des Doppelsulfats von 1-Desoxy-(5-hydroxy-tryptamino)-D-fructose und 1-Methyl-hydantoin-2-imid, dadurch gekennzeichnet, dass man 1-Methyl-hydantoin-2-imid zu einer wässrigen Lösung von 1-Desoxy-(5-hydroxy-tryptamino)-D-fructose mit einem Gehalt an Schwefelsäure bei einem pH-Wert von ungefähr 3 zusetzt und das Doppelsulfat von 1-Desoxy-(5-hydroxy-tryptamino)-D-fructose und von 1-Methyl-hydantoin-2-imid aus dem Reaktionsmilieu abtrennt.

2. Verfahren nach Anspruch 1, dadurch gekennzeichnet, dass man das Doppelsulfat von 1-Desoxy-(5-hydroxy-tryptamino)-D-fructose durch Kristallisation aus dem Reaktionsmilieu abtrennt, indem man die Lösung konzentriert und zu der konzentrierten Lösung Ethanol zusetzt.

3. Verfahren nach Anspruch 1, dadurch gekennzeichnet, dass die eingesetzte 1-Desoxy-(5-hydroxy-tryptamino)-D-fructose durch Umsetzen von überschüssiger D-Glucose mit 5-Hydroxy-tryptamin in einem wasserfreien Lösungsmittel unter inerter Atmosphäre in Gegenwart von Oxalsäure oder Ameisensäure, die dem Reaktionsmilieu einen pH-Wert von 3 bis 5 verleiht, erhalten wird.

4. Verfahren nach Anspruch 1, dadurch gekennzeichnet, dass nach dem Abtrennen des Doppelsulfats von 1-Desoxy-(5-hydroxy-tryptamino)-D-fructose das Reaktionsmilieu mit einer starken Base zur Einstellung des pH-Wertes auf den isoelektrischen Punkt des 5-Hydroxy-tryptamins behandelt wird, die letztgenannte Verbindung mit einem aliphatischen Alkohol mit 4 bis 8 Kohlenstoffatomen oder mit einem Benzylalkohol extrahiert wird, der Alkohol abgetrennt wird und das 5-Hydroxy-tryptamin in die Bildungsreaktion der 1-Desoxy-(5-hydroxy-tryptamino)-D-fructose zurückgeführt wird.

5. Verfahren nach Anspruch 1 oder 2, dadurch gekennzeichnet, dass zur Herstellung der wässrigen Lösung von 1-Desoxy-(5-hydroxy-tryptamino)-D-fructose mit einem Gehalt an Schwefelsäure zu einem die 1-Desoxy-(5-hydroxy-tryptamino)-D-fructose in Gegenwart von Wasser enthaltenden Reaktionsmilieu ein Calciumhydroxidüberschuss, bezogen auf 1-Desoxy-(5-hydroxy-tryptamino)-D-fructose, zugesetzt wird, ein unlöslicher Additionskomplex gewonnen wird, dieser mit einer das Calcium ausfällenden Säure behandelt wird, das Calcium, in Form eines unlöslichen Salzes beseitigt wird, die 1-Desoxy-(5-hydroxy-tryptamino)-D-fructose in Lösung gewonnen wird und zu der Lösung Schwefelsäure zugesetzt wird.

6. Verfahren nach Anspruch 5, dadurch gekennzeichnet, dass die das Calcium ausfällende verwendete Säure die Schwefelsäure ist.

7. Verfahren nach Anspruch 5 oder 6, dadurch gekennzeichnet, dass nach dem Ausfällen des unlöslichen Additionskomplexes der 1-Desoxy-(5-hydroxy-tryptamino)-D-fructose mit dem Calciumhydroxid dieser Komplex abgetrennt und gewaschen wird, die Mutterlaugen der Waschung aufgefangen werden, diese mit Schwefelsäure behandelt werden, bis die Lösung einen pH-Wert von ungefähr 3 aufweist, das gebildete Calciumsulfat beseitigt wird, der pH-Wert der flüssigen Phase durch Zugabe einer starken Base auf den isoelektrischen Punkt des 5-Hydroxy-tryptamins eingestellt wird, das 5-Hydroxy-tryptamin mit einem aliphatischen Alkohol mit 4 bis 8 Kohlenstoffatomen oder mit einem Benzylalkohol extrahiert wird, der Alkohol beseitigt wird und das 5-Hydroxy-tryptamin in die Bildungsreaktion der 1-Desoxy-(5-hydroxy-tryptamino)-D-fructose zurückgeführt wird.

8. Verfahren nach einem der Ansprüche 5 bis 7, dadurch gekennzeichnet, dass nach dem Abtrennen des Doppelsulfats von 1-Desoxy-(5-hydroxy-tryptamino)-D-fructose und von 1-Methyl-hydantoin-2-imid zu den Mutterlaugen Calciumhydroxid zugesetzt wird, der gebildete unlösliche Komplex abgetrennt wird und dieser zurückgeführt wird, indem er in die nächste Charge in der Calciumhydroxid-Zugabestufe aufgenommen wird.

**Claims for the Contracting States BE, DE, FR, GB, IT, LU, NL, SE**

1. The double sulfate of 1-desoxy-(5-hydroxy-tryptamino)-D-fructose and 1-methylhydantoin-2-imide.

2. A medicament containing the compound claimed in claim 1 as active principle.

3. A process for producing the compound claimed in claim 1, characterized in that 1-methylhydantoin-2-imide is added to an aqueous solution of 1-desoxy-(5-hydroxytryptamino)-D-fructose containing sulfuric acid at a pH of approximately 3 and in that the double sulfate of 1-desoxy-(5-hydroxytryptamino)-D-fructose and 1-methylhydantoin-2-imide is separated from the reaction medium.

4. A process as claimed in claim 3, characterized in that the double sulfate of 1-desoxy-(5-hydroxy-tryptamino)-D-fructose is separated from the reaction medium by crystallization by concentrating the solution and adding ethanol to the concentrated solution.

5. A process as claimed in claim 3, characterized in that the starting 1-desoxy-(5-hydroxytrypt-

amino)-D-fructose is obtained by reacting D-glucose in excess with 5-hydroxytryptamine in an ahydrous solvent in an inert atmosphere in the presence of oxalic acid or formic acid adjusting the reaction medium to a pH of from 3 to 5.

6. A process as claimed in claim 3, characterized in that, after the double sulfate of 1-desoxy-(5-hydroxytryptamino)-D-fructose has been separated, the reaction medium is treated with a strong base to adjust the pH to the isoelectric point of the 5-hydroxytryptamine, the 5-hydroxytryptamine is extracted with an aliphatic alcohol containing from 4 to 8 carbon atoms or with a benzyl alcohol, the alcohol is eliminated and the 5-hydroxytryptamine is recycled upstream of the reaction by which the 1-desoxy-(5-hydroxytryptamino)-D-fructose is formed.

7. A process as claimed in claim 3 or 4, characterized in that, to prepare the aqueous solution of 1-desoxy-(5-hydroxytryptamino)-D-fructose containing sulfuric acid, an excess of calcium hydroxide over the 1-desoxy-(5-hydroxytryptamino)-D-fructose is added to the reaction mixture containing the 1-desoxy-(5-hydroxytryptamino)-D-fructose in the presence of water, an insoluble addition complex is collected and treated with an acid which precipitates calcium, the calcium is eliminated in the form of an insoluble salt, the 1-desoxy-(5-hydroxytryptamino)-D-fructose in solution is collected and sulfuric acid is added to the solution.

8. A process as claimed in claim 7, characterized in that the calcium-precipitating acid used is sulfuric acid.

9. A process as claimed in claim 7 or 8, characterized in that, after precipitation with calcium hydroxide, the insoluble addition complex of 1-desoxy-(5-hydroxytryptamino)-D-fructose is separated and washed, the mother liquors and washing water are collected and treated with sulfuric acid until the solution has a pH of approximately 3, the calcium sulfate formed is eliminated, the pH of the liquid phase is adjusted to the isoelectric point of the 5-hydroxytryptamine by addition of a strong base, the 5-hydroxytryptamine is extracted with an aliphatic alcohol containing from 4 to 8 carbon atoms or with a benzyl alcohol, the alcohol is eliminated and the 5-hydroxytryptamine is recycled upstream of the reaction by which the 1-desoxy-(5-hydroxytryptamino)-D-fructose is formed.

10. A process as claimed in any of claims 7 to 9, characterized in that, after separation of the double sulfate of 1-desoxy-(5-hydroxytryptamino)-D-fructose and 1-methylhydantoin-2-imide, calcium hydroxide is added to the mother liquors, the insoluble complex formed is separated and recycled to the calcium hydroxide addition stage by incorporation in the following batch.

**Claims for the Contracting State AT**

1. A process for producing the double sulfate of 1-desoxy-(5-hydroxytryptamino)-D-fructose and 1-methylhydantoin-2-imide, characterized in that 1-methylhydantoin-2-imide is added to an aqueous solution of 1-desoxy-(5-hydroxytryptamino)-D-fructose containing sulfuric acid at a pH of approximately 3 and in that the double sulfate of 1-desoxy-(5-hydroxytryptamino)-D-fructose and 1-methylhydantoin-2-imide is separated from the reaction medium.

2. A process as claimed in claim 1, characterized in that the double sulfate of 1-desoxy-(5-hydroxytryptamino)-D-fructose is separated from the reaction medium by crystallization by concentrating the solution and adding ethanol to the concentrated solution.

3. A process as claimed in claim 1, characterized in that the starting 1-desoxy-(5-hydroxytryptamino)-D-fructose is obtained by reacting D-glucose in excess with 5-hydroxytryptamine in an anhydrous solvent in an inert atmosphere in the presence of oxalic acid or formic acid adjusting the reaction medium to a pH of from 3 to 5.

4. A process as claimed in claim 1, characterized in that, after the double sulfate of 1-desoxy-(5-hydroxytryptamino)-D-fructose has been separated, the reaction medium is treated with a strong base to adjust the pH to the isoelectric point of the 5-hydroxytryptamine, the 5-hydroxytryptamine is extracted with an aliphatic alcohol, containing from 4 to 8 carbon atoms or with a benzyl alcohol, the alcohol is eliminated and the 5-hydroxytryptamine is recycled upstream of the reaction by which the 1-desoxy-(5-hydroxytryptamino)-D-fructose is formed.

5. A process as claimed in claim 1 or 2, characterized in that, to prepare the aqueous solution of 1-desoxy-(5-hydroxytryptamino)-D-fructose containing sulfuric acid, an excess of calcium hydroxide over the 1-desoxy-(5-hydroxytryptamino)-D-fructose is added to the reaction mixture containing the 1-desoxy-(5-hydroxytryptamino)-D-fructose in the presence of water, an insoluble addition complex is collected and treated with an acid which precipitates calcium, the calcium is eliminated in the form of an insoluble salt, the 1-desoxy-(5-hydroxytryptamino)-D-fructose in solution is collected and sulfuric acid is added to the solution.

6. A process as claimed in claim 5, characterized in that the calcium-precipitating acid used is sulfuric acid.

7. A process as claimed in claim 5 or 6, characterized in that, after precipitation with calcium hydroxide, the insoluble addition complex of 1-desoxy-(5-hydroxytryptamino)-D-fructose is separated and washed, the mother liquors and washing water are collected and treated with sulfuric acid until the solution has a pH of approximately 3, the calcium sulfate formed is eliminated, the pH of the liquid phase is adjusted to the isoelectric point of the 5-hydroxytryptamine by addition of a strong base, the 5-hydroxytryptamine is extracted with an aliphatic alcohol containing from 4 to 8 carbon atoms or with a benzyl alcohol, the alcohol is eliminated and the 5-hydroxytryptamine is recycled upstream of the reaction by which the

1-desoxy-(5-hydroxytryptamino)-D-fructose is formed.

8. A process as claimed in any of claims 5 to 7, characterized in that, after separation of the double sulfate of 1-desoxy-(5-hydroxytryptamino)-D-fructose and 1-methyl-hydantoin-2-imide, calcium hydroxide is added to the mother liquors, the insoluble complex formed is separated and recycled to the calcium hydroxide addition stage by incorporation in the following batch.